# EUROPEAN PATENT SPECIFICATION

(11) **EP 3 129 001 B1**
(45) Date of publication and mention of the grant of the patent: **02.10.2019**
(21) Application number: 15718459.9
(22) Date of filing: 09.04.2015
(51) Int. Cl.: A61K 9/08, A61K 39/36, A61K 47/26, A61P 37/06, A61P 37/08, A61K 39/00

(54) **HSP-FREE ALLERGEN PREPARATION**
HSP-FREIE HERSTELLUNG VON ALLERGENEN
PRÉPARATION D'ALLERGÈNE SANS HSP

(30) Priority: 10.04.2014 EP 14164293
(43) Date of publication of application: 15.02.2017
(73) Proprietor: ASIT BioTech S.A., 1200 Bruxelles (BE)
(72) Inventor: LEGON, Thierry, B-3360 Bierbeek (BE); PIROTTON, Sabine, B-1070 Bruxelles (BE); PLACIER, Gael, B-4000 Liège (BE)
(74) Representative: dompatent von Kreisler Selting Werner - Partnerschaft von Patent- und Rechtsanwälten mbB
(86) International application number: PCT/EP2015/057772
(87) International publication number: WO 2015/155310

(56) References cited:
- WO-A1-2008/000783
- WO-A1-2013/011095
- WO-A2-2010/061193
- WO-A2-2012/085872
- P Oeding: "Antigenic properties of Staphylococcus aureus", Bacteriological reviews, 1 December 1960 (1960-12-01), pages 374-396, XP055201991, Retrieved from the Internet: URL:http://www.ncbi.nlm.nih.gov/pubmed/137 30303

## Description

The present invention relates to a pharmaceutical preparation according to claim 1, especially useful for treating allergy, autoimmune disease or graft rejection.

US 6,312,711 discloses a pharmaceutically or food composition intended for treating pathologies associated with graft rejection or allergic autoimmune reaction comprising the administration of a complex of a stress protein and epitopes of an antigenic structure.

WO 2013/011095 discloses a pharmaceutical preparation for subcutaneous injection comprising between 0.5 ng and 200 µg of HSP70 between 0.5 and 100 µg of fragments of an antigenic structure.

There has been a lot of research in connection with the inclusion of heat shock proteins in pharmaceutical preparations for tolerance induction, but clinical outcome is not always satisfying. There is still a need for an improvement of these preparations.

Surprisingly it has now been found that a pharmaceutical preparation comprising
- 10 to 200 µg/ml of fragments of an antigenic structure which induces allergic reaction
- 2 to 6% (w/v) mannitol
- 0.5 to 2% (w/v) trehalose
- water,
wherein the amount of heat shock proteins is less than 1 ng/ml, wherein the fragments of an antigenic structure are hydrolyzed allergens prepared by enzymatic hydrolysis of the antigenic structure, and wherein at least 70% by weight of the fragments are between 1,000 and 10,000 Da, may be used for a safe treatment with a high efficiency.

The fragments of the antigenic structure dissolved in a solution comprising mannitol and trehalose, but without the addition of heat shock proteins are on the one hand safe in administration, but on the other hand also suitable for the treatment for inducing tolerance to the related antigen.

Mannitol and trehalose have been used in prior art for the formulation of pharmaceutical preparations, but typically in the context with lyophilized products.

The pharmaceutical preparation of the present invention is not lyophilized during production, but nevertheless provides advanced stability upon storage. The pharmaceutical preparation comprises about 2 to 6% (w/v) mannitol. The suitable amount of trehalose is in an amount of 0.5 to 2% (w/v), wherein the volume is measured at 25°C.

The preparation comprises also a buffering agent, a phosphate buffer is preferred.

In one embodiment of the invention, the pharmaceutical preparation is in a form for subcutaneous injection.

The preparation of the invention is essentially free of heat shock proteins. Essentially free of heat shock proteins refers to concentration of less than 1.0 ng/ml, more preferably less than 0.5 ng/ml. The fragments of the antigenic structure are preferably prepared by enzymatic hydrolysis of the antigenic structure. Preferred forms for preparing fragments of antigenic structures. Preferred ways of obtaining hydrolyzed allergen fragments preferably free of non-protein components of the antigens are the methods described in WO 2008/000783, WO 2009/083589 and WO 2012/172037. The major steps of these methods are:
- an extraction of allergenic proteins from the source of allergens;
- a first purification step followed by a denaturation preferably with reducing and chaotropic agents
- a further purification step
- hydrolysis of the protein.

A further denaturing step may be used prior to hydrolysis.

The antigenic structures are selected from antigenic structures which induce allergic reaction. Such antigenic structures which are also referred to as allergens. Preferred allergens are natural protein allergens. Suitable examples are selected from milk allergens, venom allergens, egg allergens, weed allergens, grass allergens, grass pollen antigens, tree allergens, shrub allergens, flower allergens, grain allergens, fungi allergens, fruit allergens, berry allergens, nut allergens, seed allergens, bean allergens fish allergens, shellfish allergens, meat allergens, spices allergens, insect allergens, mite allergens, animal allergens, animal dander allergens, allergens of Hevea brasiliensis. Very preferred allergens are grass pollen allergens, peanut allergens, house dust mite allergens, ragweed allergens and Japanese cedar allergens.

In one embodiment of the invention, the pharmaceutical preparation is used in a treatment comprising at least two injections in a patient at different time points, preferably wherein the preparation is for use in a treatment comprising of 2 to 20 injections with increasing amounts of the preparation.

A further embodiment of the present invention is a vial or application device comprising 0.2 to 1.50 ml or 0.5 to 1.50 ml of the pharmaceuticals preparation of the invention.

A further embodiment of the invention is a kit comprising 2 to 20 or 2 to 30 vials or application devices, said application devices comprising the necessary amount of the pharmaceutical preparation of the invention for use in a treatment comprising injections with increasing amounts of the preparation.

The application device is a more convenient form because it avoids the diffusion of the active ingredients present on the surface of the needle in the derma.

In a typical embodiment, the application devices are syringes. For example, the application devices could comprise a solution of 100 µg/ml of the preparation and the first syringe could comprise 50 µl, other devices 100, 200, 500 µl and 1000 µl. The advantage is that the preparation is ready to use. Prefilled application devices reduce the error rate during application. Surprisingly the preparation of the present invention is stable at the temperature of a refrigerator for at least six months, preferably more than a year. Even if stored at room temperature, stability allows storage for a similar time period. These properties avoid cumbersome work related to dissolving lyophilized preparations prior to use.

It is believed that mannitol and trehalose provide for the high stability of the preparation, thus, increasing the safety and efficacy of the preparation.

A further embodiment of the present invention is a pharmaceutical preparation according to claims 1 to 6 for use in a method for treating allergy comprising administering to a patient by a subcutaneous injection, a cumulated dose of 40 to 1000 µg of fragments of an antigenic structure using the pharmaceutical preparation of the invention.

Preferred time intervals between injection sessions are 2 to 10 days.

In one preferred embodiment, the patient receives two subcutaneous injections at different loci of the patient's body during the injection session, e.g. doctor visit. The injections are preferably performed with a 30 to 60 min interval. Preferred loci for injections are the left and the right arm of a patient.

### Examples

The safety and effect of the preparation was analyzed. The study was conducted in the University Hospital Carl-Gustav-Carus, Dresden, Germany together with the University Hospital of Cologne, Cologne, Germany.

### Effect

As test material, an allergen extract from *Lolium perenne* was used comprising:
- 100 µg/ml hydrolyzed pollen allergen extracts prepared according to WO 2008/000783
- 42 mg/ml mannitol
- 10.2 mg/ml trehalose
- 0.69 mg/ml phosphate
- 0.70 mg/ml NaCl
- and water.

The administration scheme included 6 visits with 2 injections administered at a time interval of 30 min in each arm. Within the total of 12 subcutaneous injections on 6 visits, the cumulative dose was 490 µg. Before and after treatment, additional visits (visit 1, visit 8) were conducted. It was a monocentric study with 61 patients with allergic rhinitis or rhinoconjunctivitis due to grass pollen allergens. In this analysis, 44 patients were included, the others are still within the treatment.

The dosage regimen was 5 µg, 10 µg, 20 µg, 40 µg, 70 µg and 100 µg at a concentration of 100 µg/ml. As it is applied in each arm, the double amount was applied per visit. From this interim analysis of 44 patients, 38 completed the study, 6 drop-outs occurred, one was for systemic reactions, one was for private reasons and 4 patients were no longer reachable.

Including the drop-outs, the cumulative dose applied to the patients can be derived from table 1.

**Table 1:**

| **Cumulative dose [µg]** | **Frequency** | **Percentage** |
|---|---|---|
| 30 | 1 | 2.3 |
| 60 | 1 | 2.3 |
| 70 | 1 | 2.3 |
| 150 | 1 | 2.3 |
| 190 | 2 | 4.5 |
| 290 | 2 | 4.5 |
| 390 | 1 | 2.3 |
| 490 | 35 | 79.5 |
| Total | 44 | 100.0 |

Prior to the treatment, a skin prick test was conducted. The results of the skin reaction (expressed in mm) can be seen in table 2. The positive control was an injection of histamine.

**Table 2:**

| | **Negative Control** | **Positive Control** | **Grass Pollen Allergens** | **Ragweed Allergens** | **Dust mite DP* Allergens** | **Dust mite DF* Allergens** | **Cat Allergens** | **Dog Allergens** |
|---|---|---|---|---|---|---|---|---|
| N | 44 | 44 | 44 | 44 | 44 | 44 | 44 | 44 |
| Mean | 0 | 6.23 | 6.66 | 0.34 | 1.36 | 0.70 | 1.95 | 1.52 |
| Median | 0 | 6.00 | 6.00 | 0 | 0 | 0 | 0 | 0 |
| Standard Deviation | 0 | 1.70 | 2.37 | 0.96 | 2.25 | 1.67 | 2.65 | 2.03 |
| Minimum | 0 | 4 | 4 | 0 | 0 | 0 | 0 | 0 |
| Maximum | 0 | 13 | 15 | 3 | 9 | 8 | 9 | 7 |

| | | | | | | | | |
|---|---|---|---|---|---|---|---|---|
| DP: Dermatophagoides pteronyssinus DF: Dermatophagoides farinae | | | | | | | | |

Additionally, the serum level of IgE was tested. The results are reported in table 3.

**Table 3:**

| | **IgE [kU/I] Lolch Weidelgras g5** |
|---|---|
| Mean | 43.07 |
| Standard deviation | 33.94 |
| 25^{th} percentile | 13.70 |
| Median | 29.10 |
| 75^{th} percentile | 62.70 |
| Minimum | 2.4 |
| Maximum | 101.0 |

The efficacy of the experiment was tested using a diagnostic solution from ALK-Abellò named "Provokationslösung ALK-lyophilisiert Gräser". Increasing amounts were injected and the reaction was tested in a conjunctival provocation test (CPT) at visit 1, visit 6 and visit 8. The CPT stages were defined as in table 4 (Gronemeyer's grading, Richelman et al 2003 Arch. Allergy. Immunol. 130; 51-59).

**Table 4:**

| **Stage** | **Findings** |
|---|---|
| 0 | No subjective or visible reaction |
| I | Itching, reddening, foreign body sensation |
| II | Stage I and in addition tearing, vasodilatation of conjunctiva, bulbi |
| III | Stage II and in addition vasodilatation and erythema of conjunctiva tarsi, blepharospasm |
| IV | Stage III and in addition chemosis, lid swelling |

The results are reported in Tables 5 to 7.

**Table 5:**

| **Visit 1** | **Rate** | **Percentage of valid** |
|---|---|---|
| Valid | 37 | 100 |
| • No response | 1 | 2.7 |
| • Reaction at 100 SQ-E/ml | 8 | 21.6 |
| • Reaction at 1,000 SQ-E/ml | 18 | 48.6 |
| • Reaction at 10,000 SQ-E/ml | 10 | 27 |
| Missing | 7 | |
| Total | 44 | |

**Table 6:**

| **Visit 6** | **Rate** | **Percentage of valid** |
|---|---|---|
| Valid | 38 | 100 |
| • No response | 29 | 76.3 |
| • Reaction at 100 SQ-E/ml | 2 | 5.3 |
| • Reaction at 1,000 SQ-E/ml | 2 | 5.3 |
| • Reaction at 10,000 SQ-E/ml | 5 | 13.2 |
| Missing | 6 | |
| Total | 44 | |

**Table 7:**

| **Visit 8** | **Rate** | **Percentage of valid** |
|---|---|---|
| Valid | 37 | 100 |
| • No response | 24 | 64.9 |
| • Reaction at 100 SQ-E/ml | 0 | 0 |
| • Reaction at 1,000 SQ-E/ml | 1 | 2.7 |
| • Reaction at 10,000 SQ-E/ml | 12 | 32.4 |
| Missing | 7 | |
| Total | 44 | |

In summary, only one patient (2.7%) was showing no response to the "Provokationslösung" prior to treatment. This improved to 29 patients (76.3%) during treatment and 24 patients (64.9%) after treatment. An improvement of the CPT is achieved at visit 8 for 93.8% of all patients.

### Safety

24 patients did not mention any adverse effect, 20 patients had one or more adverse effects, but no one experienced a serious adverse effect. At the site of injection, typically a redness and wheal occurs at injection site. The size wheal is an important safety indicator. It is measured about 30 minutes after injection.

Table 8 shows the variation of the redness diameter in cm during the visits and injections. Surprisingly and unexpected, the typical increase of redness diameter with increasing amounts of injection did not occur. In contrast, the redness diameter was almost stable or even decreased slightly during treatment. This is unexpected and an indication of the well-tolerated administration.

**Table 8:**

| | **Mean value of the redness (cm)** | | | | | | | | | | | |
|---|---|---|---|---|---|---|---|---|---|---|---|---|
| | Visit 2 | | Visit 3 | | Visit 4 | | Visit 5 | | Visit 6 | | Visit 7 | |
| | Inj. 1 | Inj. 2 | Inj. 3 | Inj. 4 | Inj. 5 | Inj. 6 | Inj. 7 | Inj. 8 | Inj. 9 | Inj. 10 | Inj. 11 | Inj. 12 |
| Valid | 44 | 44 | 44 | 44 | 43 | 42 | 42 | 42 | 40 | 40 | 37 | 36 |
| Missing | 0 | 0 | 0 | 0 | 1 | 2 | 2 | 2 | 4 | 4 | 7 | 8 |
| Mean | 1.69 | 1.92 | 2.46 | 2.54 | 1.91 | 1.98 | 2.01 | 2.11 | 2.09 | 2.09 | 1.70 | 1.85 |
| Standard deviation | 1.40 | 1.67 | 1.21 | 1.28 | 1.25 | 1.09 | 1.12 | 1.22 | 1.76 | 1.58 | 1.65 | 1.75 |
| 25^{th} percentile | 0 | 0.4 | 1.63 | 1.63 | 1.0 | 1.0 | 1.38 | 1.0 | 0 | 1.0 | 0 | 0 |
| Median | 2.0 | 2.0 | 2.50 | 2.75 | 2.0 | 2.0 | 2.0 | 2.0 | 2.25 | 2.0 | 1.50 | 2.0 |
| 75^{th} percentile | 3.0 | 3.0 | 3.50 | 3.38 | 3.0 | 3.0 | 2.50 | 3.0 | 3.50 | 3.0 | 3.0 | 3.38 |
| Minimum | 0 | 0 | 0 | 0 | 0 | 0 | 0 | 0 | 0 | 0 | 0 | 0 |
| Maximum | 4.0 | 8.0 | 4.50 | 5.0 | 4.0 | 4.0 | 5.0 | 5.0 | 5.0 | 6.0 | 5.0 | 5.0 |

**Table 9:**

| | **Mean value of the wheal (cm) 20 to 60 min after injection** | | | | | | | | | | | |
|---|---|---|---|---|---|---|---|---|---|---|---|---|
| | Visit 2 | | Visit 3 | | Visit 4 | | Visit 5 | | Visit 6 | | Visit 7 | |
| | Inj. 1 | Inj. 2 | Inj. 3 | Inj. 4 | Inj. 5 | Inj. 6 | Inj. 7 | Inj. 8 | Inj. 9 | Inj. 10 | Inj. 11 | Inj. 12 |
| Valid | 44 | 44 | 44 | 44 | 43 | 42 | 42 | 42 | 40 | 40 | 37 | 36 |
| Missing | 0 | 0 | 0 | 0 | 1 | 2 | 2 | 2 | 4 | 4 | 7 | 8 |
| Mean | 0.36 | 0.43 | 0.56 | 0.52 | 0.49 | 0.54 | 0.52 | 0.51 | 0.58 | 0.54 | 0.56 | 0.50 |
| Standard deviation | 0.30 | 0.56 | 0.28 | 0.17 | 0.26 | 0.31 | 0.32 | 0.36 | 0.53 | 0.50 | 0.56 | 0.53 |
| 25^{th} percentile | 0 | 0 | 0.4 | 0.4 | 0.3 | 0.4 | 0.3 | 0.4 | 0.3 | 0.3 | 0.3 | 0.3 |
| Median | 0.4 | 0.4 | 0.5 | 0.5 | 0.5 | 0.5 | 0.5 | 0.45 | 0.4 | 0.4 | 0.4 | 0.4 |
| 75^{th} percentile | 0.58 | 0.5 | 0.6 | 0.68 | 0.5 | 0.53 | 0.53 | 0.6 | 0.5 | 0.5 | 0.5 | 0.5 |
| Minimum | 0 | 0 | 0.2 | 0.2 | 0 | 0 | 0 | 0 | 0 | 0.2 | 0.2 | 0.2 |
| Maximum | 1.0 | 3.5 | 2.0 | 1.0 | 1.50 | 2.0 | 2.0 | 2.50 | 2.50 | 3.0 | 3.0 | 3.50 |

Additionally, the typical wheal reactions were below 1 cm. According to guidelines, adaptation of treatment is required when the local reaction reaches the limit of about 5 cm. In the present study, the maximum value observed for a wheal was 3.5 cm.

Especially the wheal diameter is a good indication of systemic reactions.

Immediate allergic systemic reaction emerging within 30 minutes after injection were reported in few patients, the reactions were graded in accordance to the recommendations of AWMF for the management of these reactions (Ring, J., Akuttherapie anaphylaktischer Reaktionen. Leitlinie der Deutschen Gesellschaft für Allergologie und klinische Immunologie (DGAKI), des Ärzteverbandes Deutscher Allergologen (ÄDA), der Gesellschaft für Pädiatrische Allergologie und Umweltmedizin (GPA) und der Deutschen Akademie für Allergologie und Umweltmedizin (DAAU). Allergo J 2007, 16: p. 420-434). Immediate allergic systemic reactions of grade I (mild) were reported in 2 patients (3.3% of patients) and 5 immediate allergic systemic reaction of grade II (moderate) were reported in 4 patients (6.6% of patients). These frequencies are lower than the frequency reported in literature (22% for grade II systemic reactions in a meta analysis of Calderon MA, Alves B, Jacobson M, Hurwitz B, Sheikh A, Durham S Allergen injection immunotherapy for seasonal allergic rhinitis (Review) The Cochrane Library 2007, Issue 1).

### Conclusion

The treatment was well-tolerated and highly efficient.

### Comparative Study

To compare the safety of the preparation comprising mannitol/trehalose to technical phosphate buffer saline preparations. This comparative preparation comprised
- 100 µg/ml pollen peptides as used in the first study
- 0.35 mg/ml Phosphate
- 0.56 mg/ml NaCl
- 0.01 mg/ml KCl.

The studies differed in that the product had a different formulation and each treatment was splitted into two injections, i.e. more allergens was applied.

**Table 10:**

| | **Mean wheal diameters (cm) reported 8 hours after injection** | | | | | | | | | | | | |
|---|---|---|---|---|---|---|---|---|---|---|---|---|---|
| | | **Visit 1** | | **Visit 2** | | **Visit 3** | | **Visit 4** | | **Visit 5** | | **Visit 6** | |
| | | Inj. 1 | Inj. 2 | Inj. 1 | Inj. 2 | Inj. 1 | Inj. 2 | Inj. 1 | Inj. 2 | Inj. 1 | Inj. 2 | Inj. 1 | Inj. 2 |
| Comparative composition | Daily dose (µg)* | 5 | | 10 | | 20 | | 50 | | 50 | | | |
| | N | 9 | | 9 | | 9 | | 9 | | 9 | | | |
| | Mean | **3.56** | | **3.33** | | **2.39** | | **2.72** | | **2.11** | | | |
| | Median | 4.0 | | 5.0 | | 2.0 | | 2.0 | | 2.0 | | | |
| | Minimum | 0.0 | | 0.0 | | 0.0 | | 0.0 | | 0.0 | | | |
| | Maximum | 12.0 | | 9.0 | | 6.5 | | 7.5 | | 7.0 | | | |
| Inventive composition | Daily dose (µg)* | 5 | 5 | 10 | 10 | 20 | 20 | 40 | 40 | 70 | 70 | 100 | 100 |
| | N | 59 | 59 | 57 | 57 | 56 | 56 | 55 | 55 | 55 | 55 | 52 | 52 |
| | Mean | **0.41** | **0.50** | **0.62** | **0.62** | **0.57** | **0.50** | **0.63** | **0.69** | **0.51** | **0.51** | **0.59** | **0.75** |
| | Median | 0.00 | 0.00 | 0.00 | 0.00 | 0.00 | 0.00 | 0.00 | 0.00 | 0.00 | 0.00 | 0.00 | 0.00 |
| | Minimum | 0.0 | 0.0 | 0.0 | 0.0 | 0.0 | 0.0 | 0.0 | 0.0 | 0.0 | 0.0 | 0.0 | 0.0 |
| | Maximum | 4.8 | 6.8 | 6.5 | 7.5 | 7.8 | 7.3 | 9.5 | 9.0 | 7.3 | 6.3 | 7.0 | 7.0 |

| | | | | | | | | | | | | | |
|---|---|---|---|---|---|---|---|---|---|---|---|---|---|
| *theoretical daily dose; some patients may have received the next lowest dose if large reactions had been previously observed | | | | | | | | | | | | | |

Unexpectedly the use of the trehalose/mannitol composition resulted in a reduction of wheal diameter.

## Claims

1. A pharmaceutical preparation comprising
- 10 to 200 µg/ml of fragments of an antigenic structure which induces allergic reaction
- 2 to 6% (w/v) mannitol
- 0.5 to 2% (w/v) trehalose
- water,
wherein the amount of heat shock proteins is less than 1 ng/ml, wherein the fragments of an antigenic structure are hydrolyzed allergens prepared by enzymatic hydrolysis of the antigenic structure, and wherein at least 70% by weight of the fragments are between 1,000 and 10,000 Da.

2. The pharmaceutical preparation of claim 1, wherein the concentration of fragments of an antigenic structure which induce allergic reaction is 50 to 200 µg/ml.

3. The pharmaceutical preparation of claims 1 or 2 further comprising buffering agents, preferably phosphate buffer.

4. The pharmaceutical preparation of any of claims 1 to 3, wherein the pharmaceutical preparation is in a form for subcutaneous injection.

5. The pharmaceutical preparation of claim 1, wherein the hydrolyzed allergens are obtainable by a method comprising the steps of:
a) extracting a source of allergens comprising allergenic proteins to form an extract,
b) purifying the extract to remove non-protein components to form a purified extract,
c) denaturing the purified extract with a first denaturing agent to form a purified denatured extract,
d) refining the purified denatured extract to remove impurities to form a refined denatured extract,
e) denaturing the refined denatured extract with a second denaturing agent to form denatured allergen mixture, and
f) hydrolyzing the denatured allergen mixture to form hydrolyzed allergens.

6. The pharmaceutical preparation of any of claims 1 to 5, wherein the antigenic structures are milk allergens, venom allergens, egg allergens, weed allergens, grass allergens, grass pollen antigens, tree allergens, shrub allergens, flower allergens, grain allergens, fungi allergens, fruit allergens, berry allergens, nut allergens, seed allergens, bean allergens fish allergens, shellfish allergens, meat allergens, spices allergens, insect allergens, mite allergens, animal allergens, animal dander allergens and allergens of Hevea brasiliensis.

7. A preparation of any of claims 1 to 6, wherein the preparation is for use in a treatment of allergy comprising at least two injections in a patient at different time points, preferably wherein the preparation is for use in a treatment comprising of 2 to 20 injections with increasing amounts of the preparation.

8. A vial or an application device comprising 0.2 to 1.5 ml of the pharmaceuticals preparation of any one of claims 1 to 6.

9. The application device of claim 8 wherein the application devices are syringes.

10. A kit comprising 2 to 30 vials or application devices of claim 8.

11. The pharmaceutical preparation of any one of claims 1 to 6 for use in a method of treating allergy comprising administering to a patient by a subcutaneous injection, a cumulated dose of 40 µg to 1000 µg of fragments of an antigenic structure in one or more injection sessions.

12. The preparation for use according to claim 11, wherein injection sessions are performed in intervals of 3 to 10 days.

13. The preparation for use according to any one of claims 11 or 12, wherein two subcutaneous injections are performed in the same injection sessions at different loci of the patient's body, preferably
wherein the two different loci are on the arms of a patient.

14. The preparation for use according to any of claims 11 to 13, wherein the treatment is repeated every year.

## Patentansprüche

1. Pharmazeutisches Präparat, umfassend:
- 10 bis 200 µg/ml Fragmente einer antigenen Struktur, die eine allergische Reaktion induzieren;
- 2 bis 6% (w/v) Mannit;
- 0,5 bis 2% (w/v) Trehalose;
- Wasser;
wobei die Menge der Hitzeschockproteine kleiner als 1 ng/ml ist, wobei die Fragmente einer antigenen Struktur hydrolysierte Allergene sind, die durch enzymatische Hydrolyse der antigenen Struktur hergestellt werden, und wobei wenigstens 70 Gew.-% der Fragmente zwischen 1000 und 10 000 Da betragen.

2. Pharmazeutisches Präparat gemäß Anspruch 1, wobei die Konzentration von Fragmenten einer antigenen Struktur, die eine allergische Reaktion induzieren, 50 bis 200 µg/ml beträgt.

3. Pharmazeutisches Präparat gemäß Anspruch 1 oder 2, weiterhin umfassend Puffermittel, vorzugsweise Phosphatpuffer.

4. Pharmazeutisches Präparat gemäß einem der Ansprüche 1 bis 3, wobei das pharmazeutische Präparat in einer Form für subkutane Injektion vorliegt.

5. Pharmazeutisches Präparat gemäß Anspruch 1, wobei die hydrolysierten Allergene nach einem Verfahren erhältlich sind, das die folgenden Schritte umfasst:
a) Extrahieren einer Quelle von Allergenen, die allergene Proteine umfasst, unter Bildung eines Extrakts;
b) Reinigen des Extrakts zur Entfernung von Nichtproteinkomponenten unter Bildung eines gereinigten Extrakts;
c) Denaturieren des gereinigten Extrakts mit einem ersten Denaturierungsmittel unter Bildung eines gereinigten denaturierten Extrakts;
d) Raffinieren des gereinigten denaturierten Extrakts zur Entfernung von Verunreinigungen unter Bildung eines raffinierten denaturierten Extrakts;
e) Denaturieren des raffinierten denaturierten Extrakts mit einem zweiten Denaturierungsmittel unter Bildung eines denaturierten Allergengemischs; und
f) Hydrolysieren des denaturierten Allergengemischs unter Bildung von hydrolysierten Allergenen.

6. Pharmazeutisches Präparat gemäß einem der Ansprüche 1 bis 5, wobei die antigenen Strukturen Milchallergene, Tiergiftallergene, Ei-Allergene, Unkrautallergene, Grasallergene, Graspollenallergene, Baumallergene, Strauchallergene, Blumenallergene, Kornallergene, Pilzallergene, Fruchtallergene, Beerenallergene, Nussallergene, Samenallergene, Bohnenallergene, Fischallergene, Schalentierallergene, Fleischallergene, Gewürzallergene, Insektenallergene, Milbenallergene, Tierallergene, Tierhaarallergene und Allergene von *Hevea brasiliensis* sind.

7. Präparat gemäß einem der Ansprüche 1 bis 6, wobei das Präparat zur Verwendung bei einer Behandlung einer Allergie dient, die wenigstens zwei Injektionen bei einem Patienten zu unterschiedlichen Zeitpunkten umfasst, wobei vorzugsweise das Präparat zur Verwendung bei einer Behandlung dient, die 2 bis 20 Injektionen mit steigenden Mengen des Präparats umfasst.

8. Stechampulle oder Applikationsvorrichtung, die 0,2 bis 1,5 ml des pharmazeutischen Präparats gemäß einem der Ansprüche 1 bis 6 umfasst.

9. Applikationsvorrichtung gemäß Anspruch 8, wobei die Applikationsvorrichtungen Spritzen sind.

10. Kit, der 2 bis 30 Stechampullen oder Applikationsvorrichtungen gemäß Anspruch 8 umfasst.

11. Pharmazeutisches Präparat gemäß einem der Ansprüche 1 bis 6 zur Verwendung in einem Verfahren zur Behandlung einer Allergie, das das Verabreichen einer kumulierten Dosis von 40 µg bis 1000 µg Fragmenten einer antigenen Struktur an einen Patienten durch eine subkutane Injektion in einer oder mehreren Injektionssitzungen umfasst.

12. Präparat zur Verwendung gemäß Anspruch 11, wobei Injektionssitzungen in Abständen von 3 bis 10 Tagen durchgeführt werden.

13. Präparat zur Verwendung gemäß einem der Ansprüche 11 oder 12, wobei zwei subkutane Injektionen in denselben Injektionssitzungen an unterschiedlichen Stellen des Körpers des Patienten durchgeführt werden, wobei sich vorzugsweise die zwei unterschiedlichen Stellen an den Armen eines Patienten befinden.

14. Präparat zur Verwendung gemäß einem der Ansprüche 11 bis 13, wobei die Behandlung jedes Jahr wiederholt wird.

## Revendications

1. Préparation pharmaceutique comprenant
- 10 à 200 µg/mL de fragments d'une structure antigénique qui induit une réaction allergique
- 2 à 6 % (p/v) de mannitol
- 0,5 à 2 % (p/v) de tréhalose
- de l'eau,
dans laquelle la quantité de protéines de choc thermique est de moins de 1 ng/mL,
dans laquelle les fragments d'une structure antigénique sont des allergènes hydrolysés préparés par hydrolyse enzymatique de la structure antigénique, et
dans laquelle au moins 70 % en poids des fragments sont entre 1 000 et 10 000 Da.

2. Préparation pharmaceutique selon la revendication 1, dans laquelle la concentration de fragments d'une structure antigénique qui induisent une réaction allergique est de 50 à 200 µg/mL.

3. Préparation pharmaceutique selon les revendications 1 ou 2, comprenant en outre des agents tampons, de préférence un tampon de phosphate.

4. Préparation pharmaceutique selon l'une quelconque des revendications 1 à 3, dans laquelle la préparation pharmaceutique est sous une forme pour injection sous-cutanée.

5. Préparation pharmaceutique selon la revendication 1, dans laquelle les allergènes hydrolysés peuvent être obtenus par une méthode comprenant les étapes de :
a) extraction d'une source d'allergènes comprenant des protéines allergéniques pour former un extrait,
b) purification de l'extrait pour éliminer des composants non protéiques pour former un extrait purifié,
c) dénaturation de l'extrait purifié avec un premier agent de dénaturation pour former un extrait dénaturé purifié,
d) raffinage de l'extrait dénaturé purifié pour éliminer des impuretés en vue de former un extrait dénaturé raffiné,
e) dénaturation de l'extrait dénaturé raffiné avec un second agent de dénaturation pour former un mélange d'allergènes dénaturés, et
f) hydrolyse du mélange d'allergènes dénaturés pour former des allergènes hydrolysés.

6. Préparation pharmaceutique selon l'une quelconque des revendications 1 à 5, dans laquelle les structures antigéniques sont des allergènes de lait, des allergènes de venin, des allergènes d'oeuf, des allergènes d'adventice, des allergènes d'herbe, des antigènes de pollen d'herbe, des allergènes d'arbre, des allergènes d'arbuste, des allergènes de fleur, des allergènes de graine, des allergènes de champignon, des allergènes de fruit, des allergènes de baie, des allergènes de fruit à coque, des allergènes de graine, des allergènes de haricot, des allergènes de poisson, des allergènes de fruit de mer, des allergènes de viande, des allergènes d'épice, des allergènes d'insecte, des allergènes d'acarien, des allergènes d'animal, des allergènes de pellicules animales et des allergènes d'Hevea brasiliensis.

7. Préparation selon l'une quelconque des revendications 1 à 6, dans laquelle la préparation est à utiliser dans un traitement d'allergie comprenant au moins deux injections chez un patient à différents instants, de préférence dans laquelle la préparation est à utiliser dans un traitement comprenant 2 à 20 injections avec des quantités croissantes de la préparation.

8. Flacon ou dispositif d'application comprenant 0,2 à 1,5 mL de la préparation pharmaceutique de l'une quelconque des revendications 1 à 6.

9. Dispositif d'application selon la revendication 8, dans lequel les dispositifs d'application sont des seringues.

10. Nécessaire comprenant 2 à 30 flacons ou dispositifs d'application de la revendication 8.

11. Préparation pharmaceutique selon l'une quelconque des revendications 1 à 6 à utiliser dans une méthode de traitement d'allergie comprenant l'administration à un patient par une injection sous-cutanée, d'une dose cumulée de 40 µg à 1 000 µg de fragments d'une structure antigénique en une ou plusieurs sessions d'injection.

12. Préparation à utiliser selon la revendication 11, dans laquelle des sessions d'injection sont réalisées à des intervalles de 3 à 10 jours.

13. Préparation à utiliser selon l'une quelconque des revendications 11 ou 12, dans laquelle deux injections sous-cutanées sont réalisées dans les mêmes sessions d'injection à différents lieux du corps du patient, de préférence
dans laquelle les deux lieux différents sont sur les bras d'un patient.

14. Préparation à utiliser selon l'une quelconque des revendications 11 à 13, dans laquelle le traitement est répété chaque année.
